# EUROPÄISCHE PATENTSCHRIFT

(11) **EP 0 427 963 B1**
(45) Veröffentlichungstag und Bekanntmachung des Hinweises auf die Patenterteilung: **30.03.1994**
(21) Anmeldenummer: 90119673.3
(22) Anmeldetag: 15.10.1990
(51) Int. Cl.: C07C 273/18, C07C 275/28, C07D 277/82

(54) **Verfahren zur Herstellung reiner, unsymmetrisch disubstituierter Harnstoffe**
Process for the preparation of pure asymmetric disubstituted urea
Procédé pour la préparation d'urée disubstituée asymétrique pure

(30) Priorität: 10.11.1989 AT 2575/89
(43) Veröffentlichungstag der Anmeldung: 22.05.1991
(73) Patentinhaber: AGROLINZ AGRARCHEMIKALIEN GESELLSCHAFT M.B.H., A-4021 Linz (AT)
(72) Erfinder: Wörther, Rudolf Helmut, Dr., A-4020 Linz (AT); Korntner, Horst, A-4020 Linz (AT); Auer, Egmont, Dr., A-4020 Linz (AT); Thonhofer, Kurt, Dr., A-4040 Linz (AT)

(56) Entgegenhaltungen:
- GB-A- 2 170 808
- US-A- 2 673 877

## Beschreibung

Die vorliegende Erfindung betrifft ein Verfahren zur Herstellung reiner, unsymmetrisch disubstituierter Harnstoffe, ausgehend von N-Alkyl- oder N,N-Dialkylharnstoffen.

N-Alkyl- oder N,N-Dialkylharnstoffe können beispielsweise durch Umsetzung von Harnstoff mit N-Alkyl- oder N,N-Dialkylaminen nach DE-A-2937331 hergestellt werden. Solche Harnstoffe sollen nach DE-A-1064051 mit Aryl- oder Cycloalkylaminen zu unsymmetrisch disubstituierten Harnstoffen umgesetzt werden können. Wie aber schon aus dem Vergleich der dort angegebenen Schmelzpunkte mit den aus der Literatur bekannten Schmelzpunkten ersichtlicht ist, werden nach DE-A-1064051 nur Mischungen verschiedener Harnstoffe und nicht Reinprodukte erhalten.

Es wurde nun unerwarteteweise gefunden, daß reine, unsymmetrisch disubstituierte Harnstoffe hergestellt werden können, wenn man N-Alkyl- oder N,N-Dialkylharnstoffe mit einem gegebenenfalls substituierten Arylamin oder einem heterocyclischen Amin in Gegenwart jenes Amins umsetzt, das im Ausgangsprodukt, dem jeweiligen N-Alkyl- oder N,N-Dialkylharnstoff, bereits enthalten ist.

Gegenstand der Erfindung ist daher ein Verfahren zur Herstellung reiner, unsymmetrisch disubstituierter Harnstoffe der allgemeinen Formel 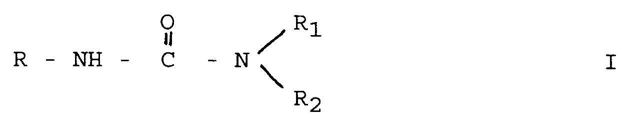
in der R einen unsubstituierten oder ein- oder mehrfach durch Halogenatome, Alkyl-, Alkoxy-, Aryloxy- oder Trifluormethylgruppen substituierten Phenylrest, einen unsubstituierten oder ein- oder mehrfach durch Halogenatome, Alkyl-, Alkoxy- oder Trifluormethylgruppen substituierten Oxazolyl-, Isoxazolyl-, Thiazolyl-, Isothiazolyl-, Benzoxazolyl- oder Benzthiazolylrest und R₁ und R₂ unabhängig voneinander ein Wasserstoffatom oder eine Alkylgruppe bedeuten, wobei R₁ und R₂ nicht gleichzeitig Wasserstoff sind oder R₁ und R₂ gemeinsam eine Butylen- oder Pentylengruppe bedeuten, durch Umsetzung eines N-Alkyl- oder N,N-Dialkylharnstoffes der allgemeinen Formel 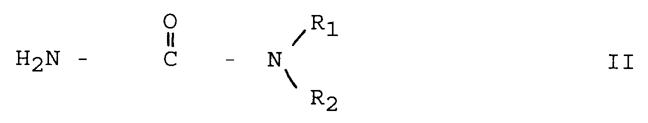
in der R₁ und R₂ die obgenannte Bedeutung haben, mit einem Amin der allgemeinen Formel

R - NH₂ III

in der R die obgenannte Bedeutung hat, gegebenenfalls in einem Verdünnungsmittel und Isolierung des Reaktionsproduktes der allgemeinen Formel I auf übliche Weise, dadurch gekennzeichnet, daß die Umsetzung in Gegenwart eines Überschusses eines Amins der allgemeinen Formel 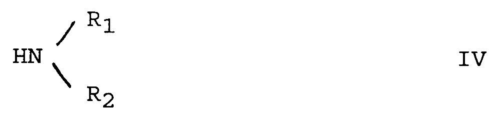
in der R₁ und R₂ jeweils die gleiche Bedeutung haben, wie im eingesetzten N-Alkyl- oder N,N-Dialkylharnstoff der allgemeinen Formel II, erfolgt.

In der allgemeinen Formel I bedeutet R einen unsubstituierten oder ein- oder mehrfach durch Halogenatome wie Fluor, Chlor oder Brom, geradkettige, verzweigte oder cyclische Alkylgruppen mit 1 bis 6 C-Atomen, wie Methyl-, Ethyl-, Propylgruppen und ihre Isomeren, wie Isopropyl-, sek. Butyl-, tert. Butylgruppen oder Cyclopropyl-, Cyclopentyl-, Cyclohexylgruppen, Alkoxygruppen mit 1 bis 5 C-Atomen, wie Methoxy-, Ethoxy-, Propoxygruppen, gegebenenfalls substituierte Aryloxygruppen wie Phenoxy-, p-Chlorphenoxy-, p-Methoxyphenoxygruppen oder Trifluormethylgruppen substituierten Phenylrest, worunter auch gemischt substituierte Phenylreste zu verstehen sind oder einen unsubstituierten oder ein- oder mehrfach durch Halogenatome, Alkyl-, Alkoxy- oder Trifluormethylgruppen substituierten Oxazolyl-, Isoxazolyl-, Thiazolyl-, Isothiazolyl-, Benzoxazolyl- oder Benzthiazolylrest, wobei als Halogenatome, als Alkyl-und Alkoxygruppen die oben angegebenen Gruppen in Frage kommen. R₁ und R₂ bedeuten unabhängig voneinander ein Wasserstoffatom oder eine geradkettige, verzweigte oder cyclische Alkylgruppe mit 1 bis 10 C-Atomen wie z. B. eine Methyl-, Ethyl-, Propyl-, Butyl-, Octylgruppe oder eine deren Isomeren wie z.B. eine Isopropyl-, sek. Butyl-, tert. Butylgruppe oder eine Cyclopentyl-, Alkylcyclopentyl-, Cyclohexyl-, Alkylcyclohexylgruppe, wobei R₁ und R₂ nicht gleichzeitig Wasserstoff sind oder R₁ und R₂ bedeuten gemeinsam eine Butylen- oder Pentylengruppe.

Falls R einen Phenylrest bedeutet, sind Verbindungen der allgemeinen Formel I bevorzugt, in denen R einen unsubstituierten oder ein- oder mehrfach durch Halogenatome, Alkyl-, Alkoxy-oder Aryloxygruppen oder Trifluormethylgruppen substituierten Phenylrest und R₁ und R₂ unabhängig voneinander ein Wasserstoffatom, eine geradkettige oder verzweigte Alkylgruppe mit 1 bis 6 C-Atomen oder eine cyclische Alkylgruppe mit 3 bis 10 C-Atomen bedeutet. Besonders bevorzugt sind dabei Verbindungen der allgemeinen Formel I, in denen R einen unsubstituierten oder ein- oder mehrfach durch Halogenatome, Alkyl-, Alkoxy-, Aryloxy oder Trifluormethylgruppen substituierten Phenylrest und R₁ und R₂ unabhängig voneinander ein Wasserstoffatom oder eine geradkettige Alkylgruppe mit 1 bis 4 C-Atomen bedeuten.

Falls R eines der oben angeführten heterocyclischen Amine bedeutet, sind Verbindungen der allgemeinen Formel I bevorzugt, in denen R einen unsubstituierten oder wie oben angegeben substituierten Isoxazolyl- oder Benzthiazolylrest bedeutet. Besonders bevorzugt sind dabei Verbindungen, in denen R einen unsubstituierten oder wie oben angegeben substituierten Benzthiazolylrest, ganz besonders bevorzugt einen unsubstituierten oder durch Alkoxygruppen substituierten Benzthiazolylrest bedeutet.

Bevorzugt sind unsymmetrisch disubstituierte Harnstoffe, die als herbizide oder fungizide Wirkstoffe im Pflanzenschutz eingesetzt werden. Solche Verbindungen sind beispielsweise der Fachwelt unter den Namen Fenuron, Siduron, Monuron, Diuron, Neburon, Chlortoluron, Metoxuron, Isoproturon, Chloroxuron, Difenoxuron, Fluometuron, Benzthiazuron,Isouron bekannt.

Zur Durchführung des Verfahrens wird ein N-Alkyl- oder N,N-Dialkylharnstoff der allgemeinen Formel II (Harnstoff II), beispielsweise herstellbar nach DE-A-2937331, mit einem Amin der allgemeinen Formel III (Amin III), in Gegenwart eines Amins der allgemeinen Formel IV (Amin IV) umgesetzt. Im Amin IV besitzen R₁ und R₂ jeweils die gleiche Bedeutung wie im eingesetzten Harnstoff II.

Die Umsetzung erfolgt bei Temperaturen von etwa 130 bis 250 °C in Gegenwart eines Verdünnungsmittels oder auch in Schmelze. Bevorzugt erfolgt die Reaktion in einem Verdünnungsmittel. Als Verdünnungsmittel kommen unter Reaktionsbedingungen inerte, organische Verdünnungsmittel in Betracht. Beispiele solcher Verdünnungsmittel sind etwa Amide wie Dimethylformamid, Dimethylacetamid, Ketone, wie Cyclohexanon, aliphatische und aromatische, gegebenenfalls chlorierte Kohlenwasserstoffe, wie Dekalin, Xylol, Tetralin oder Chlorbenzole, wie Monochlorbenzol, Dichlorbenzole, Trichlorbenzole. Bevorzugt sind Chlorbenzole, besonders bevorzugt sind Trichlorbenzole.

Der Harnstoff II wird mit einem Amin III gegebenenfalls in einem Verdünnungsmittel vorgelegt. Er kann dabei äquimolar zum Amin III oder in einem Überschuß vorgelegt werden, wird aber bevorzugt in einem Überschuß vorgelegt. Besonders bevorzugt werden pro Mol Harnstoff II 0,5 bis 0,7 Mol Amin III eingesetzt. Die Reaktionsmischung wird gerührt und erhitzt, wobei das Amin IV zugegeben wird. Mit der Zugabe des Amins IV kann bei Raumtemperatur begonnen werden, bevorzugt wird das Amin IV aber erst bei einer Temperatur, die höher ist als Raumtemperatur, zugegeben. Besonders bevorzugt wird mit der Zugabe des Amins IV bei Temperaturen von 60 bis 120 °C begonnen. Erfolgt die Reaktion in der Schmelze, kann das Amin IV auch erst zugegeben werden, wenn die Reaktionsmischung schon flüssig ist.
Das Amin IV kann gasförmig, flüssig oder fest zugegeben werden. Amine, die bei Raumtemperatur gasförmig sind, können als solche oder in einem der o.a. Verdünnungsmittel gelöst in das Reaktionsgemisch eingebracht werden. Amine, die bei Raumtemperatur nicht gasförmig sind, können gegebenenfalls durch Erhitzen vor der Zugabe in den gasförmigen Zustand übergeführt und gasförmig zugegeben werden, sie können aber auch als solche oder in einem der o.a. Verdünnungsmittel gelöst zugegeben werden. Feste Amine können beispielsweise mit Hilfe einer Förderschnecke zugegeben werden. Flüssige oder gelöste Amine werden zugetropft oder über eine Düse eingebracht, gasförmige Amine auf übliche Weise eingeleitet. Wird das Amin IV vor der Zugabe gelöst, wird vorzugsweise jenes Verdünnungsmittel verwendet, in dem die Reaktion durchgeführt wird. Das Amin IV wird dabei in einem Überschuß bezogen auf den Harnstoff II eingesetzt. Vorzugsweise werden pro Mol des Harnstoffes II 2 bis 15, besonders bevorzugt 2 bis 10 Mol des Amins IV zugegeben.
Das Amin IV wird nicht auf einmal zugegeben, sondern im Verlauf der Reaktion kontinuierlich zugesetzt. Während der Zugabe des Amins IV wird die Reaktionsmischung auf etwa 130 bis 250 °C, bevorzugt auf 160 bis 225 °C, erhitzt.
Im allgemeinen läuft die Reaktion unter Normaldruck ab, obgleich man auch unter Druck arbeiten kann, wobei Drücke bis zu 20 bar angewendet werden können.
Das bei der Reaktion entstehende Ammoniak wird auf übliche Weise abgeführt. Bei Verwendung eines gasförmigen Amins IV entsteht eine Mischung aus Ammoniak und überschüssigem Amin IV, die abgeführt wird und auf übliche Weise getrennt werden kann.

Das abgetrennte Amin IV kann erneut in die Reaktion eingesetzt werden.
Nach beendeter Reaktion wird die Reaktionsmischung abgekühlt. Wurde die Reaktion in der Schmelze durchgeführt, kristallisiert das Reaktionsprodukt der allgemeinen Formel I dabei aus.
Wurde die Reaktion in einem Verdünnungsmittel durchgeführt, wird der gegebenenfalls ausgefallene Niederschlag abfiltriert oder das Verdünnungsmittel wird abdestilliert. Das jeweils erhaltene Rohprodukt kann auf üblich Art und Weise gereinigt werden.
Bevorzugt wird das Rohprodukt zur Reinigung in Wasser aufgekocht und die Mischung abgekühlt. Der Niederschlag wird abgesaugt und getrocknet. Im allgemeinen ist die Reinheit des auf diese Weise gereinigten Produktes ausgezeichnet. Für spezielle Zwecke kann aber noch eine weitere Reinigung, etwa durch Umkristallisieren oder Chromatographie angeschlossen werden.

In einer bevorzugten Ausführungsform wird N,N-Dimethylharnstoff in Trichlorbenzol mit einem Amin III, worin R einen unsubstituierten oder substituierten Phenylrest bedeutet unter Rühren vorgelegt und auf 40 bis 100 °C erhitzt. Hierauf wird kontinuierlich Dimethylamin eingeleitet, wobei das Reaktionsgemisch weiter auf etwa 180 bis 220 °C erhitzt wird. Nach beendeter Reaktion wird auf Raumtemperatur abgekühlt und der gegebenenfalls ausgefallene Niederschlag abfiltriert oder das Verdünnungsmittel wird abdestilliert. Das jeweils erhaltene Rohprodukt wird auf übliche Weise vom Verdünnungsmittel befreit und mit Wasser aufgekocht. Die Mischung wird abgekühlt, der Niederschlag abgesaugt und getrocknet.

In einer besonders bevorzugten Ausführungsform wird der Harnstoff II durch Umsetzen von Harnstoff mit einem Amin IV etwa nach DE-A-2937331 in jenem Reaktionsgefäß hergestellt, in dem anschließend die Reaktion gemäß Anspruch 1 durchgeführt wird, ohne daß der Harnstoff II isoliert wird.

Es hat sich als besonders zweckmäßig herausgestellt, die beim Abfiltieren der Reaktionsprodukte vom verwendeten Verdünnungsmittel anfallenden Mutterlaugen immer wieder für eine neuerliche Reaktion zur Herstellung der gleichen Reaktionsprodukte einzusetzen, da die Ausbeuten dadurch hoch ansteigen, wobei die Reinheit der Reaktionsprodukte unerwarteterweise gleich gut bleibt.

Mit Hilfe des erfindungsgemäßen Verfahrens fallen die Produkte der allgemeinen Formel I in hervorragender Reinheit und guten Ausbeuten an. Das Verfahren stellt somit eine Bereicherung der Technik dar.

### Beispiel 1

### N-(4-Isopropylphenyl)-N′,N′-dimethylharnstoff

3,38 g (0,025 Mol) 4-Isopropylanilin wurden mit 3,35 g (0,038 Mol) N,N-Dimethylharnstoff in 20 ml 1,2,4-Trichlorbenzol unter Rühren versetzt. Die Reaktionsmischung wurde auf 205 °C erhitzt, wobei ab 120 °C Dimethylamin eingeleitet wurde. Nach 5,5 Stunden waren 8,8, g (0,19 Mol) Dimethylamin eingeleitet und die Reaktion beendet.
Die Reaktionsmischung wurde auf Raumtemperatur abgekühlt, wobei ein Niederschlag ausfiel. Der Niederschlag wurde abfiltriert, mit 1,2,4-Trichlorbenzol gewaschen, getrocknet und anschließend in 100 ml Wasser aufgekocht. Die wäßrige Mischung wurde abgekühlt, der Niederschlag abgesaugt und getrocknet. Dabei wurden 4,0 g N-(4-Isopropylphenyl)-N′,N′-dimethylharnstoff, das entspricht 78 % der Theorie, mit einem Schmelzpunkt von 155 bis 156 °C und einer Reinheit von 98,7 % erhalten.

### Beispiel 1a

Unter Verwendung der gleichen Menge 4-Isopropylanilin und N,N-Dimethylharnstoff wie im Beispiel 1, jedoch unter Verwendung der nach Beispiel 1 erhaltenen Mutterlauge anstatt des reinen 1,2,4-Trichlorbenzols als Verdünnungsmittel wurden auf gleiche Art und Weise wie in Beispiel 1 4,55 g N-(4-Isopropylphenyl)-N′,N′-dimethylharnstoff, das sind 88 % der Theorie mit einem Schmelzpunkt von 155 bis 157 °C und einer Reinheit von 97 % erhalten.

### Beispiel 1b

Wurde wie Beispiel 1a, jedoch unter Verwendung der Mutterlauge aus Beispiel 1a als Verdünnungsmittel ausgeführt.
Ausbeute: 4,75 g, das enspricht 92 % der Theorie
Schmelzpunkt: 155 - 157 °C
Reinheit: 100 %

### Beispiel 1c

Wurde wie Beispiel 1b, jedoch unter Verwendung der Mutterlauge aus Beispiel 1b als Verdünnungsmittel ausgeführt.
Ausbeute: 4,6 g, das entspricht 89 % der Theorie
Schmelzpunkt: 155 - 157 °C
Reinheit: 99,9 %

### Beispiel 1d

Wurde wie Beispiel 1c, jedoch unter Verwendung der Mutterlauge aus Beispiel 1c als Verdünnungsmittel ausgeführt.
Ausbeute: 4,65 g, das entspricht 90 % der Theorie
Schmelzpunkt: 155 - 157 °C
Reinheit: 98,3 %

### Beispiel 1e

Wurde wie Beispiel 1d, jedoch unter Verwendung der Mutterlauge aus Beispiel 1d als Verdünnungsmittel ausgeführt.
Ausbeute: 4,6 g, das entspricht 89 % der Theorie
Schmelzpunkt: 155 - 157 °C
Reinheit: 99,2 %

### Beispiel 2

### N-(4-Bromphenyl)-N′,N′-dimethylharnstoff

4,3 g (0,025 Mol) 4-Bromanilin wurden mit 3,35 g (0,038 Mol) N,N-Dimethylharnstoff in 28 ml 1,2,4-Trichlorbenzol unter Rühren versetzt, worauf die Mischung langsam auf 205 °C erhitzt wurde. Ab 50 °C wurde Dimethylamin eingeleitet. Nach 5,5 Stunden waren 11,6 g (0,25 Mol) Dimethylamin eingeleitet, und die Reaktion beendet.
Die Reaktionsmischung wurde auf Raumtemperatur abgekühlt, wobei ein Niederschlag ausfiel, der abfiltriert, mit 1,2,4-Trichlorbenzol gewaschen, bei 100 °C im Vakuum getrocknet und anschließend in 100 ml Wasser aufgekocht wurde. Die wäßrige Mischung wurde abgekühlt, der Niederschlag abgesaugt und getrocknet. Dabei wurden 4,73 g N-(4-Bromphenyl)-N′,N′-dimethylharnstoff, das sind 78 % der Theorie, mit einem Schmelzpunkt von 168 bis 170 °C erhalten.

### Beispiel 3

### N-(4-Chlorphenyl)-N′,N′-dimethylharnstoff

Unter Verwendung von 3,19 g (0,025 Mol) 4-Chloranilin, 3,35 g (0,038 Mol) N,N-Dimethylharnstoff und 10,8 g (0,23 Mol) Dimethylamin wurden auf die im Beispiel 2 beschriebene Art und Weise 4,05 g N-(4-Chlorphenyl)-N′,N′-dimethylharnstoff, das sind 82 % der Theorie, mit einem Schmelzpunkt von 170 bis 173 °C und einer Reinheit von 100 % erhalten.

### Beispiel 3a

### N-(4-Chlorphenyl)-N′,N′-dimethylharnstoff

Auf die im Beispiel 3 beschriebene Art und Weise und unter Verwendung der gleichen Mengen 4-Chloranilin und Dimethylharnstoff, jedoch unter Verwendung der im Beispiel 3 erhaltenen Mutterlauge anstatt des reinen 1,2,4-Trichlorbenzols als Verdünnungsmittel und einer Menge von 12,8 g (0,28 Mol) Dimethylamin wurden 4,45 g N-(4-Chlorphenyl)-N′,N′-dimethylharnstoff, das sind 90 % der Theorie, mit einem Schmelzpunkt von 170 bis 173 °C und einer Reinheit von 99 % erhalten.

### Beispiel 4

### N-(3-Trifluormethylphenyl)-N′,N′-dimethylharnstoff

Unter Verwendung von 4,03 g (0,025 Mol) 3-Trifluormethylanilin, 3,35 g (0,038 Mol) N,N-Dimethylharnstoff und 13,1 g (0,28 Mol) Dimethylamin wurden auf die im Beispiel 2 beschriebene Art und Weise 4,35 g N-(3-Trifluomethylphenyl)-N′,N′-dimethylharnstoff, das sind 75 % der Theorie mit einem Schmelzpunkt von 160 bis 161 °C und einer Reinheit von 99,2 %, erhalten.

### Beispiel 4a

### N-(3-Trifluormethylphenyl)-N′,N′-dimethylharnstoff

Unter Verwendung der im Beispiel 4 angegebenen Menge von 3-Trifluormethylanilin und N,N-Dimethylharnstoff jedoch unter Einsatz der Mutterlauge aus Beispiel 4 anstatt des reinen 1,2,4-Trichlorbenzols als Verdünnungsmittel und unter Verwendung von 11,4 g (0,25 Mol) Dimethylamin wurden auf die im Beispiel 2 beschriebene Art und Weise 4,8 g N-(3-Trifluormethylphenyl)-N′,N′-dimethylharnstoff, das sind 83 % der Theorie, mit einem Schmelzpunkt von 160 bis 161 °C und einer Reinheit von 99,7 % erhalten.

### Beispiel 5

### N-(3-Chlor-4-methylphenyl)-N′,N′-dimethylharnstoff

Unter Verwendung von 3,54 g (0,025 Mol) 3-Chlor-4-methylanilin, 3,35 g (0,038 Mol) N,N-Dimethylharnstoff und 14,5 g (0,31 Mol) Dimethylamin wurden auf die im Beispiel 2 beschriebene Art und Weise 3,85 g N-(3-Chlor-4-methylphenyl)-N′,N′-dimethylharnstoff, das sind 72 % der Theorie mit einem Schmelzpunkt von 142 bis 147 °C und einer Reinheit von 99,3 % erhalten.

### Beispiel 6

### N-(3,4-Dichlorphenyl)-N′,N′-dimethylharnstoff

Unter Verwendung von 4,05 g (0,025 Mol) 3,4-Dichloranilin, 3,35 g (0,038 Mol) N,N-Dimethylharnstoff und 11,0 g (0,24 Mol) Dimethylamin wurden auf die im Beispiel 2 beschriebene Art und Weise 4,7 g N-(3,4-Dichlorphenyl)-N′,N′-dimethylharnstoff, das sind 81 % der Theorie mit einer Schmelzpunkt von 153 bis 156 °C und einer Reinheit von 99,4 % erhalten.

### Beispiel 7

### N-Phenyl-N′,N′-dimethylharnstoff

2,33 g (0.025 Mol) Anilin wurden mit 3,35 g (0,038 Mol) N,N-Dimethylharnstoff in 28 ml 1,2,4-Trichlorbenzol unter Rühren versetzt. Die Reaktionsmischung wurde langsam auf 205 °C erhitzt, wobei ab 60 °C Dimethylamin eingeleitet wurde. Nach 5,5 Stunden waren 11,8 g (0,27 Mol) Dimethylamin eingeleitet und die Reaktion beendet. Die Reaktionsmischung wurde auf Raumtemperatur abgekühlt, wobei ein Niederschlag ausfiel. Der Niederschlag wurde abfiltriert, mit 1,2,4-Trichlorbenzol gewaschen, getrocknet und anschließend in Wasser aufgekocht. Die wäßrige Mischung wurde abgekühlt, der Niederschlag abgesaugt und getrocknet.
Dabei wurden 3,3 g N-Phenyl-N′,N′-dimethylharnstoff, das sind 81 % der Theorie, mit einem Schmelzpunkt von 130 bis 133 °C erhalten.

### Beispiel 8

### N-(4-Methoxyphenyl)-N′,N′-dimethylharnstoff

Unter Verwendung von 3,08 g (0,025 Mol) 4-Methoxyanilin, 3,35 g (0,038 Mol) N,N-Dimethylharnstoff und 15,0 g (0,34 Mol) Dimethylamin wurden auf die im Beispiel 7 beschriebene Art und Weise 3,1 g N-(4-Methoxyphenyl)-N′,N′-dimethylharnstoff, das sind 64 % der Theorie, mit einem Schmelzpunkt von 126 bis 130 °C erhalten.

### Beispiel 9

### N-(Benzthiazolyl-2)-N′,N′-dimethylharnstoff

Unter Verwendung von 3,76 g (0,025 Mol) 2-Aminobenzthiazol, 3,35 g (0,038 Mol) N,N-Dimethylharnstoff und 15,7 g (0,36 mol) Dimethylamin, wobei die Einleitzeit 4 Stunden betrug, wurden auf die im Beispiel 7 beschriebene Art und Weise 4,2 g N-(Benzthiazolyl-2)-N′,N′-dimethylharnstoff, das sind 76 % der Theorie mit einem Schmelzpunkt von 214 bis 216 °C erhalten.

### Beispiel 10

### N-(6-Ethyloxybenzthiazolyl-2)-N′,N′-dimethylharnstoff

Unter Verwendung von 4,86 g (0,025 Mol) 2-Amino-6-ethoxybenzthiazol, 3,35 g (0,038 Mol) N,N-Dimethylharnstoff und 13,3 g (0,30 Mol) Dimethylamin wurden auf die im Beispiel 7 beschriebene Art und Weise 3,7 g N-(6-Ethoxybenzthiazolyl-2)-N′,N′-dimethylharnstoff, das sind 56 % der Theorie mit einem Schmelzpunkt von 183 bis 186 °C erhalten.

### Bespiel 11

### N-(4-Ethoxyphenyl)-N′,N′-diethylharnstoff

3,43 g (0,025 Mol) 4-Ethoxyanilin und 4,41 g (0,038 Mol) N,N-Diethylharnstoff in 25 ml 1,2,4-Trichlorbenzol wurden unter Rühren auf 205 °C erhitzt. Ab 60 °C wurde eine Mischung von 5,5 g (0,075 Mol) Diethylamin und 3 ml 1,2,4-Trichlorbenzol innerhalb von 5,5 Stunden der Reaktionslösung zugesetzt. Das Lösungsmittel wurde abgedampft und der kristallisierende Rückstand in Wasser aufgekocht, die Mischung abgekühlt, filtiert und getrocknet. Dabei wurden 4,2 g N-(4-Ethoxyphenyl)-N′,N′-diethylharnstoff, das sind 76 % der Theorie mit einem Schmelzpunkt von 95 bis 98 °C erhalten.

### Beispiel 12

### N-(4-Chlorphenyl)-N′-methylharnstoff

3,19 g 95%iges 4-Chloranilin (0,025 Mol) wurden mit 2,82 g (0,038 Mol) N-Methylharnstoff in 28 ml 1,2,4-Trichlorbenzol versetzt. Die Mischung wurde unter Rühren langsam auf 205 °C erhitzt, wobei ab 60 °C Methylamin in die Mischung eingeleitet wurde. Nach 5,5 Stunden waren 15,2 g (0,35 Mol) Methylamin eingeleitet und die Reaktion beendet. Das Lösungsmittel wurde abgedampft und das erhaltenen Rohprodukt über eine Kieselgelsäule (Merck 9385, Eluationsmittel Tetrahydrofuran : Cyclohexan 2 : 1) gereinigt.
Dabei wurden 3,2 g N-(4-Chlorphenyl)-N′-methylharnstoff, das sind 69 % der Theorie mit einem Schmelzpunkt von 204 bis 206 °C erhalten. Nach Umkristallisieren aus Ethanol : Wasser = 7 : 3 wurde ein Schmelzpunkt von 206 bis 208 °C erhalten.

### Beispiel 13

### N-(4-Chlorphenyl)-N′-butyl-N′-methylharnstoff

3,19 g (0,025 Mol) 4-Chloranilin prakt. wurden mit 4,95 g (0,038 Mol) N-Butyl-N-methylharnstoff in 25 ml 1,2,4-Trichlorbenzol vermischt und unter Rühren langsam auf 205 °C erhitzt. Ab 100 °C wurde eine Lösung von 6,6 g (0,075 Mol) N-Butyl-N-methylamin in 3 ml 1,2,4-Trichlorbenzol innerhalb von 4 Stunden in die Reaktionsmischung eingetragen. Die Reaktionsmischung wurde 4 Stunden auf dieser Temperatur gehalten und dann auf 20 °C abgekühlt, wobei ein Niederschlag ausfiel. Der ausgefallene Niederschlag wurde abfiltriert, mit 1,2,4-Trichlorbenzol und Petrolether gewaschen und im Vakuum bei 80 °C getrocknet. Das erhaltene Rohprodukt wurde mit 60 ml Wasser aufgekocht, auf 20 °C abgekühlt, der Niederschlag abgesaugt, mit Wasser gewaschen und bei 70 °C im Vakuum getrocknet.
Dabei wurden 2,9 g N-(4-Chlorphenyl)-N′-butyl-N′-methylharnstoff, das sind 48 % der Theorie, mit einem Schmelzpunkt von 115 bis 117 °C erhalten.

### N-Butyl-N-methylharnstoff

18 g (0,3 Mol) Harnstoff wurden in 50 ml Xylol suspendiert. Die Suspension wurde auf 130 bis 135 °C erhitzt und unter Rühren mit einer Lösung von 26,15 g (0,3 Mol) N-Butyl-N-methylamin in 25 ml Xylol versetzt. Hierauf wurde auf halbes Volumen abgedampft und gekühlt, wobei ein Niederschlag ausfiel. Der Niederschlag wurde abfiltriert, mit wenig Xylol gewaschen und im Vakuum bei 70 °C getrocknet.
Dabei wurden 27,3 g N-Butyl-N-methylharnstoff, das sind 70 % der Theorie, mit einem Schmelzpunkt von 84 bis 88 °C erhalten.

### Beispiel 14

### N-(4-(4-Chlorphenoxy)phenyl)-N′,N′-dimethylharnstoff

5,49 g (0,025 Mol) 4-Amino-4′-chlor-diphenylether wurden mit 3,35 g (0,038 Mol) N,N-Dimethylharnstoff in 28 ml 1,2,4-Trichlorbenzol unter Rühren versetzt. Die Reaktionsmischung wurde auf 205 °C erhitzt, wobei ab 60 °C Dimethylamin eingeleitet wurde. Nach 5,5 Stunden waren 15,1 g (0,33 Mol) Dimethylamin eingeleitet und die Reaktion beendet.
Die Reaktionsmischung wurde auf Raumtemperatur abgekühlt, wobei ein Niederschlag ausfiel. Der Niederschlag wurde abfiltriert, mit 1,2,4-Trichlorbenzol gewaschen, getrocknet und anschließend in Wasser aufgekocht. Die wäßrige Mischung wurde abgekühlt, der Niederschlag abgesaugt und getrocknet. Dabei wurden 5,75 g N-(4-(4-Chlorphenoxy)phenyl)-N′,N′-dimethylharnstoff, das entspricht 79 % der Theorie, mit einem Schmelzpunkt von 150 bis 152 °C erhalten.

## Patentansprüche

1. Verfahren zur Herstellung reiner, unsymmetrisch disubstituierter Harnstoffe der allgemeinen Formel 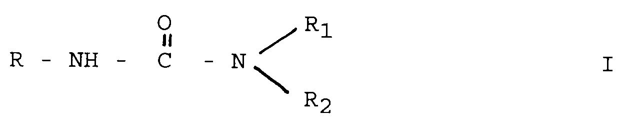 in der R einen unsubstituierten oder ein- oder mehrfach durch Halogenatome, Alkyl-, Alkoxy-, Aryloxy- oder Trifluormethylgruppen substituierten Phenylrest, einen unsubstituierten oder ein- oder mehrfach durch Halogenatome, Alkyl-, Alkoxy- oder Trifluormethylgruppen substituierten Oxazolyl-, Isoxazolyl-, Thiazolyl-, Isothiazolyl-, Benzoxazolyl- oder Benzthiazolylrest und R₁ und R₂ unabhängig voneinander ein Wasserstoffatom oder eine Alkylgruppe bedeuten, wobei R₁ und R₂ nicht gleichzeitig Wasserstoff sind oder R₁ und R₂ gemeinsam eine Butylen- oder Pentylengruppe bedeuten, durch Umsetzung eines N-Alkyl- oder N,N-Dialkylharnstoffes der allgemeinen Formel 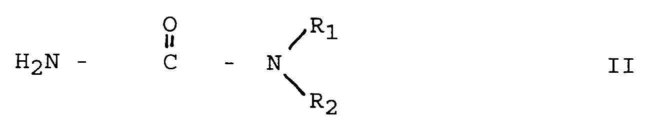 in der R₁ und R₂ die obgenannte Bedeutung haben, mit einem Amin der allgemeinen Formel
R - NH₂ III
in der R die obgenannte Bedeutung hat, gegebenenfalls in einem Verdünnungsmittel und Isolierung des Reaktionsproduktes der allgemeinen Formel I auf übliche Weise, dadurch gekennzeichnet, daß die Umsetzung in Gegenwart eines Überschusses eines Amins der allgemeinen Formel 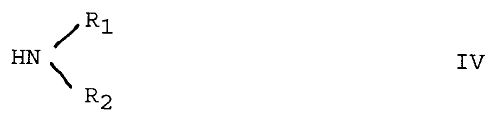 in der R₁ und R₂ jeweils die gleiche Bedeutung haben, wie im eingesetzten N-Alkyl- oder N,N-Dialkylharnstoff der allgemeinen Formel II, erfolgt.

2. Verfahren nach Anspruch 1, dadurch gekennzeichnet, daß der N-Alkyl- oder N,N-Dialkylharnstoff der allgemeinen Formel II durch Umsetzung von Harnstoff mit einem Amin der allgemeinen Formel IV gegebenenfalls in einem Verdünnungsmittel hergestellt und ohne Isolierung zum unsymmetrisch disubstituierten Harnstoff der allgemeinen Formel I umgesetzt wird.

3. Verfahren nach einem der Ansprüche 1 oder 2, dadurch gekennzeichnet, daß die Umsetzung in einem Verdünnungsmittel durchgeführt wird.

4. Verfahren nach Anspruch 3, dadurch gekennzeichnet, daß die Umsetzung in Trichlorbenzol durchgeführt wird.

5. Verfahren nach einem der Ansprüche 1 bis 4, dadurch gekennzeichnet, daß die Mutterlauge, die nach der Isolierung des Reaktionsproduktes der allgemeinen Formel I nach Anspruch 1 anfällt, in einer weiteren Umsetzung zur Herstellung des gleichen Reaktionsproduktes der allgemeinen Formel I als Verdünnungsmittel eingesetzt wird.

6. Verfahren nach einem der Ansprüche 1 bis 5, dadurch gekennzeichnet, daß ein N-Alkyl - oder N,N-Dialkylharnstoff der allgemeinen Formel II und ein Amin der allgemeinen Formel IV, worin R₁ und R₂ jeweils unabhängig voneinander Alkylgruppen mit 1 bis 4 C-Atomen bedeuten, wobei R₁ und R₂ in Formel II und in Formel IV dieselbe Bedeutung haben, eingesetzt wird.

7. Verfahren nach Anspruch 6, dadurch gekennzeichnet, daß als N-Alkyl- oder N,N-Dialkylharnstoff Dimethylharnstoff und als Amin der allgemeinen Formel IV Dimethylamin eingesetzt wird.

8. Verfahren nach einem der Ansprüche 1 bis 7, dadurch gekennzeichnet, das ein Amin der allgemeinen Formel III, in der R einen unsubstituierten oder einen durch Halogenatome, Alkyl- oder Alkoxygruppen ein- oder mehrfach substituierten Phenylrest bedeutet, eingesetzt wird.

9. Verfahren nach einem der Ansprüche 1 bis 8, dadurch gekennzeichnet, daß pro Mol des N-Alkyl- oder N,N-Dialkylharnstoffes der allgemeinen Formel II 2 bis 15 Mol des Amins der allgemeinen Formel IV eingesetzt werden.

10. Verfahren nach einem der Ansprüche 1 bis 9, dadurch gekennzeichnet, das pro Mol des N-Alkyl- oder N,N-Dialkylharnstoffes der allgemeinen Formel II 0,5 bis 0,7 Mol des Amins der allgemeinen Formel III eingesetzt werden.

## Claims

1. Process for the preparation of pure, unsymmetrically disubstituted ureas of the general formula 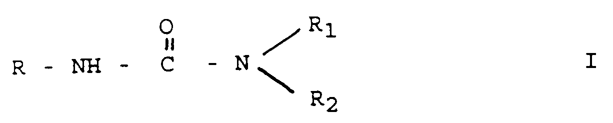 in which R denotes a phenyl radical which is unsubstituted, or monosubstituted or polysubstituted by halogen atoms or alkyl, alkoxy, aryloxy or trifluoromethyl groups, an oxazolyl, isoxazolyl, thiazolyl, isothiazolyl, benzoxazolyl or benzothiazolyl radical which is unsubstituted, or monosubstituted or polysubstituted by halogen atoms or alkyl, alkoxy or trifluoromethyl groups and R₁ and R₂ independently of one another denote a hydrogen atom or an alkyl group, where R₁ and R₂ are not simultaneously hydrogen or R₁ and R₂ together denote a butylene or pentylene group, by reacting an N-alkyl- or N,N-dialkylurea of the general formula 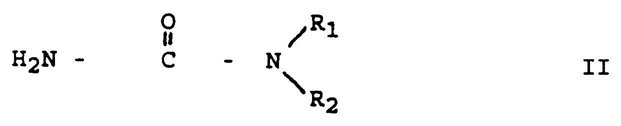 in which R₁ and R₂ have the abovementioned meaning, if desired in a diluent, with an amine of the general formula
R - NH₂ III
in which R has the abovementioned meaning, and isolating the reaction product of the general formula I in a customary manner, characterized in that the reaction is carried out in the presence of an excess of an amine of the general formula 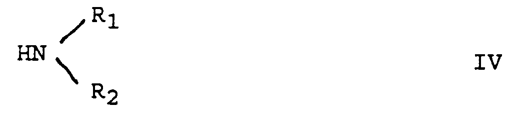 in which R₁ and R₂ in each case have the same meaning as in the N-alkyl- or N,N-dialkylurea employed of the general formula II.

2. Process according to Claim 1, characterized in that the N-alkyl- or N,N-dialkylurea of the general formula II is prepared by reaction of urea with an amine of the general formula IV, if desired in a diluent, and reacted without isolation to give the unsymmetrically disubstituted urea of the general formula I.

3. Process according to one of Claims 1 or 2, characterized in that the reaction is carried out in a diluent.

4. Process according to Claim 3, characterized in that the reaction is carried out in trichlorobenzene.

5. Process according to one of Claims 1 to 4, characterized in that the mother liquor which is obtained after the isolation of the reaction product of the general formula I according to Claim 1 is employed as the diluent in a further reaction for the preparation of the same reaction product of the general formula I.

6. Process according to one of Claims 1 to 5, characterized in that an N-alkyl- or N,N-dialkylurea of the general formula II and an amine of the general formula IV in which R₁ and R₂ in each case independently of one another denote alkyl groups having 1 to 4 C atoms is employed, R₁ and R₂ in formula II and in formula IV having the same meaning.

7. Process according to Claim 6, characterized in that dimethylurea is employed as the N-alkyl- or N,N-dialkylurea and dimethylamine is employed as the amine of the general formula IV.

8. Process according to one of Claims 1 to 7, characterized in that an amine of the general formula III in which R denotes a phenyl radical which is unsubstituted, or monosubstituted or polysubstituted by halogen atoms, or alkyl or alkoxy groups is employed.

9. Process according to one of Claims 1 to 8, characterized in that 2 to 15 mol of the amine of the general formula IV are employed per mol of the N-alkyl-or N,N-dialkylurea of the general formula II.

10. Process according to one of Claims 1 to 9, characterized in that 0.5 to 0.7 mol of the amine of the general formula III is employed per mol of the N-alkyl-or N,N-dialkylurea of the general formula II.

## Revendications

1. Procédé de fabrication d'urées pures à disubstitution asymétrique de la formule générale 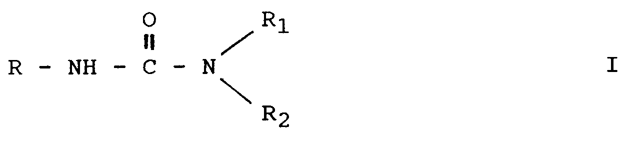 dans laquelle R représente un groupe phényle nonsubstitué ou un groupe phényle avec substitution d'un ou plusieurs atomes par des atomes d'halogène, des groupes alkyles, alkoxy, aryloxy ou trifluorométhyl, un groupe oxazolyl, isoxazolyl, thiazolyl, isothiazolyl, benzoxazolyl ou benzthiazolyl nonsubstitué ou avec substitution d'un ou plusieurs atomes par des atomes d'halogène, des groupes alkyles, alkoxy, aryloxy ou trifluorométhyl, où R₁ et R₂ signifient indépendamment l'un de l'autre un atome d'hydrogène ou un groupe alkyle et où R₁ et R₂ ne sont pas simultanément de l'hydrogène, ou encore où R₁ et R₂ constituent ensemble un groupe butylène ou pentylène par transposition d'une urée N-alkyle ou N,N-dialkyle de la formule générale 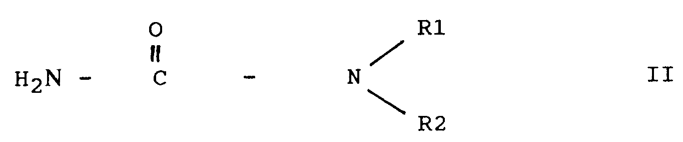 dans laquelle R1 et R2 ont la signification mentionnée ci-dessus, avec une amine de la formule générale
R - NH₂ III
dans laquelle R a la signification mentionnée ci-dessus, le cas échéant dans un diluant et isolation du produit réactionnel de la formule générale I selon une méthode habituelle, caractérisé par le fait que la transposition a lieu en présence d'un excédent d'une amine de la formule générale 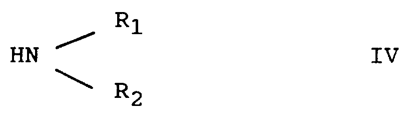 dans laquelle R1 et R2 ont respectivement la même signification que dans l'urée N-alkyle ou N,N-dialkyle de la formule générale II mise en oeuvre.

2. Procédé selon revendication 1 caractérisé par le fait que l'urée N-alkyle ou N, N-dialkyle de la formule générale II est réalisée par transposition d'urée avec une amine de la formule générale IV, le cas échéant dans un diluant, et transposée en l'urée asymétrique disubstituée sans qu'il y ait isolation.

3. Procédé selon l'une des revendications 1 ou 2 caractérisé par le fait que la transposition s'effectue dans un diluant.

4. Procédé selon revendication 3, caractérisé par le fait que la transposition s'effectue dans du trichlorobenzène.

5. Procédé selon l'une des revendications 1 à 4, caractérisé par le fait que la lessive-mère obtenue après isolation du produit réactionnel de la formule générale I selon revendication 1 est mise en oeuvre en tant que diluant lors d'une transposition ultérieure en vue de la fabrication du même produit réactionnel de la formule générale I.

6. Procédé selon l'une des revendications 1 à 5, caractérisé par le fait qu'une urée N-alkyle ou N,N-dialkyle de la formule générale II et une amine de la formule générale IV, R₁ et R₂ signifiant respectivement et indépendamment l'un de l'autre un groupe alkyle comprenant 1 à 4 atomes C, R₁ et R₂ ayant la même signification dans la formule II et dans la formule IV sont mises en oeuvre.

7. Procédé selon revendication 6, caractérisé par le fait que l'on met en oeuvre comme urée N-alkyle ou N,N-dialkyle la diméthylurée, et comme amine de la formule générale IV de la diméthylamine.

8. Procédé selon l'une des revendications 1 à 7, caractérisé par le fait que l'on met en oeuvre une amine de la formule générale III, dans laquelle R signifie un groupe phényle non-substitué ou substitué par un ou plusieurs atomes d'halogène, des groupes alkyle ou alkoxy.

9. Procédé selon l'une des revendications 1 à 8, caractérisé par le fait que, pour un mole de l'urée N-alkyle ou N,N-dialkyle de la formule générale II, 2 à 15 moles de l'amine de la formule générale IV sont mis en oeuvre.

10. Procédé selon l'une des revendications 1 à 9, caractérisé par le fait que, pour un mole de l'urée N-alkyle ou N,N-dialkyle de la formule générale II, 0,5 à 0,7 moles de l'amine de la formule générale III sont mis en oeuvre.
